# EUROPEAN PATENT SPECIFICATION

(11) **EP 2 724 658 B1**
(45) Date of publication and mention of the grant of the patent: **01.11.2017**
(21) Application number: 12855490.4
(22) Date of filing: 25.09.2012
(51) Int. Cl.: A61B 1/00, A61B 1/04, A61B 5/00

(54) **ENDOSCOPE DEVICE**
ENDOSKOPVORRICHTUNG
DISPOSITIF ENDOSCOPIQUE

(30) Priority: 07.12.2011 JP 2011268190
(43) Date of publication of application: 30.04.2014
(73) Proprietor: Olympus Corporation, Tokyo 192-8507 (JP)
(72) Inventor: TAKEI, Shunji, Hachioji-shi, Tokyo 192-8507 (JP)
(74) Representative: Schicker, Silvia
(86) International application number: PCT/JP2012/074566
(87) International publication number: WO 2013/084566

(56) References cited:
- JP-A- H10 201 707
- JP-A- 2010 068 925
- JP-A- 2011 177 436
- JP-A- 2011 177 436
- US-A1- 2008 239 070
- US-A1- 2010 245 552
- US-A1- 2010 245 616

## Description

### Technical Field

The present invention relates to an endoscope apparatus, and more particularly, to an endoscope apparatus that can observe fluorescence emitted from an intravital fluorescent substance.

### Background Art

There is a conventionally known technique that receives return light generated upon radiation of excitation light for generating fluorescence from a fluorescent substance such as a fluorescent probe dispensed beforehand to an intravital site to be observed and reference light reflected at the site to be observed, and acquires a fluorescent image as an image that makes it possible to visually distinguish the presence or absence of a lesion at the site to be observed and a reference light image (reflected light image) as an image that makes it possible to visually recognize a mucous membrane structure of the site to be observed.

To be more specific, Japanese Patent Application Laid-Open Publication No. 2011-143154 discloses an electronic endoscope system configured to receive return light generated upon radiation of illuminating light which is discretely distributed in each wavelength region of a green color region, a red color region and an infrared region where a fluorescent labeling substance dispensed beforehand into the body of a patient can be excited, and acquire an infrared fluorescent image of a focus where the fluorescent labeling substance is accumulated and an image of a biological tissue surface mainly composed of red and green colors.

Incidentally, according to the configuration in Japanese Patent Application Laid-Open Publication No. 2011-143154, in order to receive the fluorescence in the infrared region emitted from the fluorescent labeling substance, a special color filter in which an IR color filter that transmits light in the infrared region is arranged in place of a B color filter in a general RGB color filter of a Bayer array is attached to a front light receiving surface of a solid image pickup device.

Therefore, according to the configuration in Japanese Patent Application Laid-Open Publication No. 2011-143154, because of the use of the special color filter as mentioned above, there arise problems that a cost of manufacturing an apparatus such as an endoscope is increased and versatility with respect to observation of light in wavelength regions other than the fluorescence in the infrared region is flowered.

US 2008/239070 A1 discloses a device according to the preamble of claim 1. The present invention has been made in view of the forgoing circumstances and an object of the present invention is to provide an endoscope apparatus capable of performing a fluorescence observation with a configuration of low cost and having high versatility as compared with a conventional one.

### Disclosure of Invention

### Means for Solving the Problem

An endoscope apparatus according to the invention is defined in the appended claims.

### Brief Description of the Drawings

Fig. 1 is a diagram illustrating a configuration of main components of an endoscope apparatus according to an embodiment of the present invention;
Fig. 2 is a diagram illustrating an example of optical characteristics of an R filter, a G filter and a B filter provided for a color filter;
Fig. 3 is a diagram illustrating an example of optical characteristics of an excitation light cut filter;
Fig. 4 is a diagram illustrating an example of wavelength bands of R light, G light and B light emitted from a light source apparatus; and
Fig. 5 is a diagram illustrating an example of a wavelength band of return light impinging upon a scope in a fluorescence observation mode.

### Best Mode for Carrying Out the Invention

Hereinafter, an embodiment of the present invention will be described with reference to the accompanying drawings.

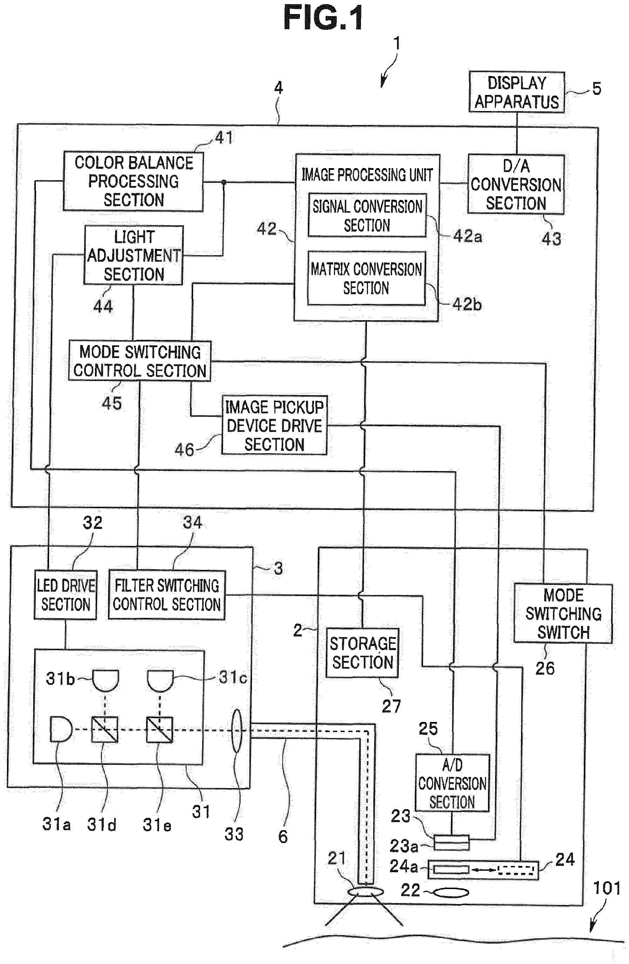

Fig. 1 to Fig. 5 relate to an embodiment of the present invention. Fig. 1 is a diagram illustrating a configuration of main components of an endoscope apparatus according to an embodiment of the present invention.

As shown in Fig. 1, an endoscope apparatus 1 includes a scope 2 configured to be insertable into a body cavity of a subject, to pick up an image of an object such as a living tissue located in the body cavity and acquire image data, a light source apparatus 3 configured to supply illuminating light emitted to the object to the scope 2, a processor 4 configured to generate and output a video signal corresponding to the image data acquired by the scope 2, and a display apparatus 5 configured to display an image corresponding to a video signal outputted from the processor 4. Furthermore, a light guide 6 configured to transmit the light supplied from the light source apparatus 3 to a distal end portion of the scope 2 is inserted in the scope 2.

The scope 2 is configured as an endoscope provided with, for example, an elongated insertion portion and includes, at a distal end portion thereof, an illumination optical system 21 that radiates illuminating light transmitted by the light guide 6 onto an object, an objective optical system 22 that forms an image of return light from the object illuminated with the illuminating light, an image pickup device 23 whose image pickup surface is placed at an image forming position of the objective optical system 22, a color filter 23a attached to the image pickup surface of the image pickup device 23, and a filter switching apparatus 24 placed in an optical path between the objective optical system 22 and the color filter 23a.

Furthermore, the scope 2 includes an A/D conversion section 25 that converts an analog image pickup signal outputted according to the image of the object picked up by the image pickup device 23 to digital image data and outputs the digital image data, a mode switching switch 26 that can issue a command relating to switching of an observation mode of the endoscope apparatus 1 and a storage section 27 that stores predetermined information to be used for image processing by the processor 4 beforehand.

The image pickup device 23 is configured to be driven based on an image pickup device drive signal outputted from the processor 4, to thereby pick up an image of an object, generate an image pickup signal corresponding to the picked up image of the object and output the image pickup signal to the A/D conversion section 25.

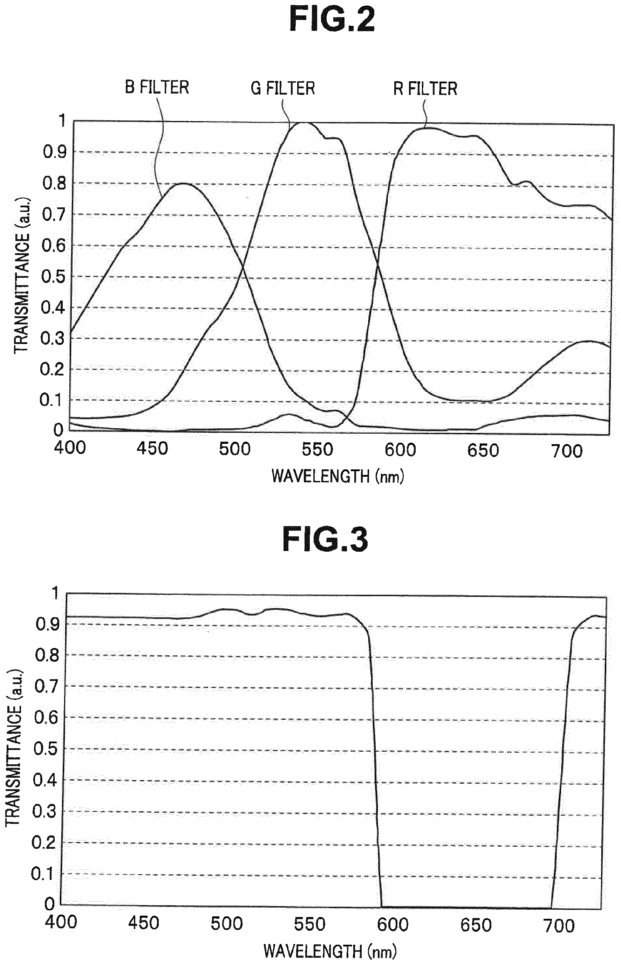

The color filter 23a is formed by arranging a plurality of filters: R (red) filters, G (green) filters and B (blue) filters, respectively provided with predetermined optical characteristics (spectral characteristics) in a Bayer array (in a checkerboard pattern) at positions corresponding to their respective pixels of the image pickup device 23. Note that in the present embodiment, suppose, for example, that the color filter 23a is provided with the R filter, the G filter and the B filter having optical characteristics shown in Fig. 2. Fig. 2 is a diagram illustrating an example of optical characteristics of the R filter, the G filter and the B filter provided for the color filter.

The R filter of the color filter 23 a is configured such that transmittance in a range from a red color region to a near-infrared region becomes relatively higher than transmittances of the other wavelength bands (see Fig. 2). That is, the R filter of the color filter 23 a is configured such that transmittances in the wavelength bands of R light and FL light which will be described later become relatively higher than transmittances of the other wavelength bands.

The G filter of the color filter 23a is configured such that transmittance in a green color region becomes relatively higher than transmittances of the other wavelength bands (see Fig. 2). That is, the G filter of the color filter 23a is configured such that transmittances in the wavelength bands of G light and REF light which will be described later become relatively higher than transmittances of the other wavelength bands.

The B filter of the color filter 23a is configured such that transmittance in a blue color region becomes relatively higher than transmittances of the other wavelength bands (see Fig. 2). That is, the B filter of the color filter 23a is configured such that transmittance in the wavelength bands of B light becomes relatively higher than transmittances of the other wavelength bands.

The filter switching apparatus 24 is configured to retract the excitation light cut filter 24a from an optical path between the objective optical system 22 and the color filter 23a upon detecting that the endoscope apparatus 1 has been switched to a white light observation mode based on a filter switching signal outputted from the light source apparatus 3. On the other hand, when the excitation light cut filter 24a is retracted from the optical path between the objective optical system 22 and the color filter 23a, the filter switching apparatus 24 is configured to transmit light in each wavelength band incident via the objective optical system 22 to the color filter 23a side.

On the other hand, upon detecting that the endoscope apparatus 1 has been switched to a fluorescence observation mode based on a filter switching signal outputted from the light source apparatus 3, the filter switching apparatus 24 is configured to perform operation of inserting the excitation light cut filter 24a into the optical path between the objective optical system 22 and the color filter 23a. When the excitation light cut filter 24a is inserted into the optical path between the objective optical system 22 and the color filter 23a, the filter switching apparatus 24 is configured to transmit only light in a predetermined wavelength band corresponding to the optical characteristics of the excitation light cut filter 24a out of the light in each wavelength band incident via the objective optical system 22 to the color filter 23a side. Fig. 3 is a diagram illustrating an example of optical characteristics of the excitation light cut filter.

The excitation light cut filter 24a is configured so as to have optical characteristics (spectral characteristics) of cutting off R light as shown in Fig. 3, for example, (transmittance of R light becomes substantially 0) and substantially transmitting light in wavelength bands other than R light.

The mode switching switch 26 is configured to be able to issue a command for switching the observation mode of the endoscope apparatus 1 to any one selected from the white light observation mode and the fluorescence observation mode according to operation by an operator or the like.

The storage section 27 made up of a non-volatile memory or the like includes a pre-stored matrix as predetermined information to be used for image processing by the processor 4. Furthermore, the storage section 27 is configured to output the aforementioned matrix to the processor 4 upon detecting that the scope 2 is connected to the processor 4. Note that details of the matrix stored in the storage section 27 will be described later.

Note that according to the present embodiment, the image pickup device 23, the color filter 23a and the A/D conversion section 25 may be configured as individual circuits or devices, or may be configured as one device such as a color CMOS sensor.

The light source apparatus 3 includes an LED light source section 31, an LED drive section 32, a condensing optical system 33 that condenses light emitted from the LED light source section 31 and supplies the condensed light to the light guide 6, and a filter switching control section 34 that outputs a filter switching signal to cause the filter switching apparatus 24 to perform operation corresponding to a mode switching signal outputted from the processor 4.

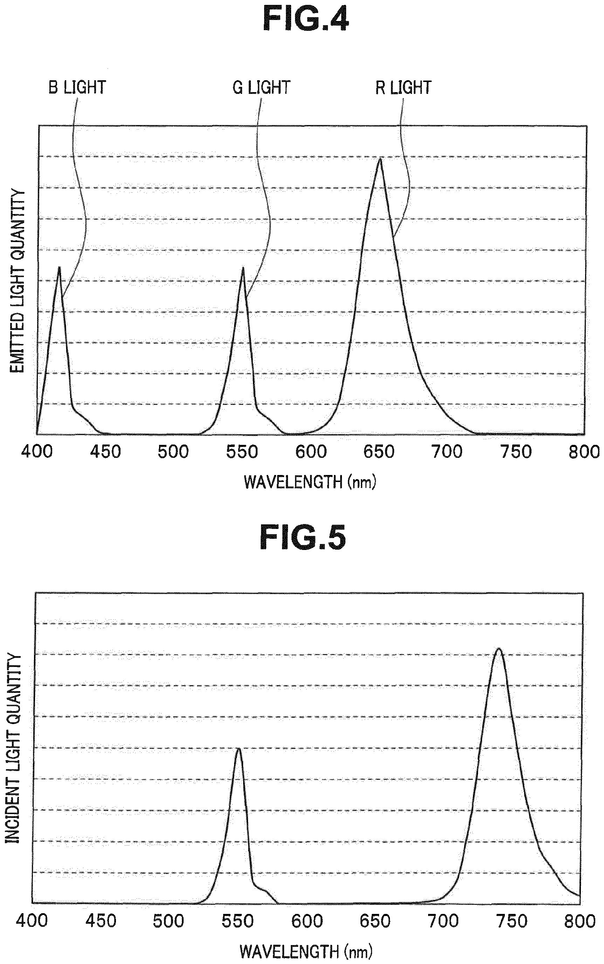

The LED light source section 31 is configured by including an LED 31a that emits light in a red color region, an LED 31b that emits light in a green color region, an LED 31c that emits light in a blue color region, an optical element 31d and an optical element 31e. Fig. 4 is a diagram illustrating an example of wavelength bands of R light, G light and B light emitted from the light source apparatus.

The LED 31a is configured to emit narrow band R light whose center wavelength is set, for example, to near 650 nm (see Fig. 4).

The LED 31b is configured to emit narrow band G light whose center wavelength is set, for example, to near 550 nm (see Fig. 4).

The LED 31c is configured to emit narrow band B light whose center wavelength is set, for example, to near 415 nm (see Fig. 4).

That is, the above configurations of the LEDs 31a, 31b and 31c are set such that the respective narrow wavelength bands of light of R, G and B colors do not overlap each other (have mutually different discrete wavelength bands).

The optical element 31d is made up of, for example, a half mirror and has such optical characteristics that the R light emitted from the LED 31a is transmitted to the optical element 31e side and the G light emitted from the LED 31b is reflected toward the optical element 31e side.

The optical element 31e is made up of, for example, a half mirror and has such optical characteristics that the R light and the G light emitted via the optical element 31d are transmitted to the condensing optical system 33 side and the B light emitted from the LED 31c is reflected toward the condensing optical system 33 side.

The LED drive section 32 is configured to be able to supply a drive current to drive each LED provided for the LED light source section 31. Furthermore, the LED drive section 32 is configured to be able to change the magnitude of the drive current supplied from the LED drive section 32 to the LED light source section 31 based on a light adjustment signal outputted from the processor 4 to thereby change the intensity (light quantity) of the light (R light, G light and B light) emitted from each LED of the LED light source section 31. Moreover, the LED drive section 32 is further configured to be able to cause each LED provided for the LED light source section 31 to turn on or off based on the light adjustment signal outputted from the processor 4.

Upon detecting that the endoscope apparatus 1 has been switched to the white light observation mode based on the mode switching signal outputted from the processor 4, the filter switching control section 34 outputs a filter switching signal to the filter switching apparatus 24 so as to operate to cause the excitation light cut filter 24a to retract from the optical path between the objective optical system 22 and the color filter 23a. Furthermore, upon detecting that the endoscope apparatus 1 has been switched to the fluorescence observation mode based on the mode switching signal outputted from the processor 4, the filter switching control section 34 outputs a filter switching signal to the filter switching apparatus 24 so as to operate to cause the excitation light cut filter 24a to be inserted into the optical path between the objective optical system 22 and the color filter 23a.

The processor 4 includes a color balance processing section 41 configured to perform processing to adjust a signal intensity balance among respective color components included in image data acquired from the scope 2, an image processing unit 42 configured to apply image processing to the image data outputted from the color balance processing section 41, a D/A conversion section 43 configured to convert the image data outputted from the image processing unit 42 to an analog video signal and output the analog video signal, a light adjustment section 44 configured to output a light adjustment signal corresponding to brightness of the image data outputted from the color balance processing section 41, a mode switching control section 45 configured to output a mode switching signal corresponding to an instruction issued from the mode switching switch 26, and an image pickup device drive section 46 that outputs an image pickup device drive signal for performing control relating to image pickup operation of the image pickup device 23.

The image processing unit 42 is configured to include functions that can execute processing such as noise correction, gamma correction and edge enhancement.

Furthermore, the image processing unit 42 is configured to perform processing by a signal conversion section 42a and a matrix conversion section 42b upon detecting that the endoscope apparatus 1 has been switched to the fluorescence observation mode based on a mode switching signal outputted from the mode switching control section 45.

The signal conversion section 42a performs processing of converting each color component included in the image data outputted from the color balance processing section 41 to a luminance component Y, and color difference components Cr and Cb.

The matrix conversion section 42b applies a matrix outputted from the storage section 27 of the scope 2 to the luminance component Y, and the color difference components Cr and Cb obtained as the processing result of the signal conversion section 42a, carries out calculations and further performs processing of allocating the image data of each color component obtained as a result of the calculations to the R channel, the G channel and the B channel of the display apparatus 5. Note that details of such processing of the matrix conversion section 42b will be described later.

Upon detecting that the endoscope apparatus 1 has been switched to the fluorescence observation mode based on the mode switching signal outputted from the mode switching control section 45, the image processing unit 42 applies processing such as noise correction, gamma correction, and edge enhancement to the image data allocated to each color channel of R, G and B of the display apparatus 5 through the processing by the matrix conversion section 42b and outputs the image data to the D/A conversion section 43.

On the other hand, upon detecting that the endoscope apparatus 1 has been switched to the white light observation mode based on the mode switching signal outputted from the mode switching control section 45, the image processing unit 42 allocates each color component included in the image data outputted from the color balance processing section 41 to each color channel of R, G and B of the display apparatus 5, further applies processing such as noise correction, gamma correction and edge enhancement to the image data allocated to each color channel and outputs the image data to the D/A conversion section 43. That is, according to the present embodiment, when the endoscope apparatus 1 is switched to the white light observation mode, the processing by the signal conversion section 42a and the matrix conversion section 42b is not performed.

Upon detecting that the endoscope apparatus 1 has been switched to the white light observation mode based on the mode switching signal outputted from the mode switching control section 45 and the image data outputted from the color balance processing section 41, the light adjustment section 44 outputs a light adjustment signal for causing each of the LED 31a, the LED 31b and the LED 31c to simultaneously emit light at the intensity appropriate for observation in the white light observation mode to the LED drive section 32. Furthermore, upon detecting that the endoscope apparatus 1 has been switched to the fluorescence observation mode based on the mode switching signal outputted from the mode switching control section 45 and the image data outputted from the color balance processing section 41, the light adjustment section 44 outputs a light adjustment signal for causing the LED 31c to turn off and the LED 31a and the LED 31b to simultaneously emit light at the intensity appropriate for observation in the fluorescence observation mode to the LED drive section 32.

Upon detecting that the mode switching switch 26 has issued an instruction for switching the observation mode of the endoscope apparatus 1 to the white light observation mode, the mode switching control section 45 outputs a mode switching signal to the filter switching control section 34, the image processing unit 42, the light adjustment section 44 and the image pickup device drive section 46 so as to perform operation corresponding to the white light observation mode. On the other hand, upon detecting that the mode switching switch 26 has issued an instruction for switching the observation mode of the endoscope apparatus 1 to the fluorescence observation mode, the mode switching control section 45 outputs a mode switching signal to the filter switching control section 34, the image processing unit 42, the light adjustment section 44 and the image pickup device drive section 46 so as to perform operation corresponding to the fluorescence observation mode.

Based on the mode switching signal outputted from the mode switching control section 45, the image pickup device drive section 46 outputs an image pickup device drive signal to the LED drive section 32 so as to perform image pickup operation at timing corresponding to the observation mode of the endoscope apparatus 1 and generate an image pickup signal using a gain corresponding to the observation mode of the endoscope apparatus 1.

Here, operation of the endoscope apparatus 1 of the present embodiment will be described.

First, operation or the like when the observation mode of the endoscope apparatus 1 has been switched to the white light observation mode will be described.

A user such as an operator connects the respective components of the endoscope apparatus 1, operates the mode switching switch 26 at timing before and after turning on power to the respective components of the endoscope apparatus 1 to thereby issue an instruction for switching the observation mode of the endoscope apparatus 1 to the white light observation mode.

Upon detecting that the mode switching switch 26 has issued an instruction for switching the observation mode of the endoscope apparatus 1 to the white light observation mode, the mode switching control section 45 outputs a mode switching signal to the filter switching control section 34, the image processing unit 42, the light adjustment section 44 and the image pickup device drive section 46 so as to perform operation corresponding to the white light observation mode.

Based on the light adjustment signal outputted from the light adjustment section 44, the LED drive section 32 causes the LED 31a, the LED 31b and the LED 31c of the LED light source section 31 to simultaneously emit light.

Through such operation of the LED drive section 32, in the white light observation mode, illuminating light (white color light) including wavelength bands of R light, G light and B light supplied from the light source apparatus 3 is emitted to an object via the light guide 6 and the illumination optical system 21 and reflected light of the R light, the G light and the B light emitted to the object is impinged on the objective optical system 22 as return light from the site to be observed 101.

On the other hand, the filter switching apparatus 24 operates so as to cause the excitation light cut filter 24a to retract from the optical path between the objective optical system 22 and the color filter 23a based on the filter switching signal outputted from the filter switching control section 34.

Through such operation of the filter switching apparatus 24, in the white light observation mode, return light (reflected light) of the R light, return light (reflected light) of the G light and return light (reflected light) of the B light that have passed through the color filter 23 a are received on the image pickup surface of the image pickup device 23 and an image pickup signal obtained by picking up image of the received light is outputted from the image pickup device 23.

The A/D conversion section 25 converts the analog image pickup signal outputted from the image pickup device 23 to digital image data and outputs the digital image data to the color balance processing section 41 of the processor 4. Through such processing of the A/D conversion section 25, image data is generated which contains a red color component RC, a green color component GC and a blue color component BC corresponding to the intensity of the R light, the G light and the B light received on the image pickup surface of the image pickup device 23.

The color balance processing section 41 applies processing for adjusting a balance of signal intensity among the RC, GC and BC color components contained in the image data (e.g., white balance processing) to the image data outputted from the A/D conversion section 25 and outputs the image data to the image processing unit 42.

Upon detecting that the endoscope apparatus 1 has been switched to the white light observation mode based on the mode switching signal outputted from the mode switching control section 45, the image processing unit 42 allocates the RC, GC and BC color components contained in the image data outputted from the color balance processing section 41 to the R, G and B color channels of the display apparatus 5, further applies processing such as noise correction, gamma correction and edge enhancement to the image data allocated to the respective color channels and outputs the image data to the D/A conversion section 43.

The display apparatus 5 displays the image of the object corresponding to the video signal outputted via the D/A conversion section 43.

That is, the operation or the like described above is performed in the white light observation mode, an observed image (color image) corresponding to the white light observation mode is thereby displayed on the display apparatus 5.

Next, operation or the like performed when the observation mode of the endoscope apparatus 1 is switched to the fluorescence observation mode will be described. Note that the following description will be given on the assumption that before observation of the site to be observed 101 in the fluorescence observation mode starts, a fluorescent probe (fluorescent substance) is dispensed to a subject (site to be observed 101) as a fluorescent probe (fluorescent substance) accumulated in a lesioned tissue such as a cancer which has an excitation wavelength in a red color region and has a fluorescence wavelength in a near-infrared region (e.g., near 700 nm) that does not overlap with the wavelength band of the G light.

The user performs operation of inserting the scope 2 while observing the observed image in the white light observation mode displayed on the display apparatus 5 and thereby places the distal end portion of the scope 2 in the vicinity of the desired site to be observed 101 in the subject. The user or the like operates the mode switching switch 26 in such a condition and thereby issues an instruction for switching the observation mode of the endoscope apparatus 1 to the fluorescence observation mode.

Upon detecting that the mode switching switch 26 has issued an instruction for switching the observation mode of the endoscope apparatus 1 to the fluorescence observation mode, the mode switching control section 45 outputs a mode switching signal to the filter switching control section 34, the image processing unit 42, the light adjustment section 44 and the image pickup device drive section 46 so as to perform operation corresponding to the fluorescence observation mode.

Based on the light adjustment signal outputted from the light adjustment section 44, the LED drive section 32 outputs a light adjustment signal to the LED drive section 32 so as to cause the LED 31c to turn off and cause the LED 31a and the LED 31b of the LED light source section 31 to simultaneously emit light.

Through such operation of the LED drive section 32, in the fluorescence observation mode, illuminating light having wavelength bands of the R light and the G light supplied from the light source apparatus 3 is emitted to the site to be observed 101 via the light guide 6 and the illumination optical system 21.

Here, because the fluorescent probe having an excitation wavelength in a red color region is dispensed to the subject (site to be observed 101), the R light emitted from the illumination optical system 21 acts as excitation light and the G light emitted from the illumination optical system 21 acts as reference light. For that reason, in the fluorescence observation mode, mixed light of the FL light which is fluorescence having a wavelength band in a near-infrared region and the REF light which is reflected light having a wavelength band of the G light is impinged on the objective optical system 22 as return light from the site to be observed 101 (see Fig. 5). Fig. 5 is a diagram illustrating an example of a wavelength band of return light impinging on the scope in the fluorescence observation mode.

That is, in the present embodiment, the wavelength band of the reference light (G light) is set to be closer to the short wavelength side than the wavelength band of the excitation light (R light). Furthermore, in the present embodiment, the color filter 23 a provided with the R filter, the G filter and the B filter having optical characteristics as shown in Fig. 2 is provided on the image pickup surface of the image pickup device 23, and the pixel at which image detection sensitivity of the FL light image included in the return light becomes maximum is thereby made to differ from the pixel at which image detection sensitivity of the REF light image included in return light becomes maximum.

Note that the present embodiment is not limited to a configuration using the G light as reference light, but may also be configured so as to use, for example, the B light as reference light or may be configured so as to use mixed light of the G light and the B light as reference light. In the case where reference light including the B light is used, it is possible to generate an observed image in which capillary vessels located on a surface layer of the site to be observed 101 can be easily visually recognized compared to the case using reference light composed of only the G light.

On the other hand, the filter switching apparatus 24 operates so as to insert the excitation light cut filter 24a into the optical path between the objective optical system 22 and the color filter 23 a based on the filter switching signal outputted from the filter switching control section 34.

Through such operation of the filter switching apparatus 24, in the fluorescence observation mode, image pickup signals acquired by picking up images of the light that has passed through the excitation light cut filter 24a and the R filter of the color filter 23 a, the light that has passed through the excitation light cut filter 24a and the G filter of the color filter 23 a and the light received on the image pickup surface of the image pickup device 23 are outputted from the image pickup device 23.

The A/D conversion section 25 converts the analog image pickup signal outputted from the image pickup device 23 to digital image data and outputs the digital image data to the color balance processing section 41 of the processor 4. Through such processing of the A/D conversion section 25, image data is generated which includes a red color component RD, a green color component GD and a blue color component BD in accordance with the intensity of the FL light and the REF light received on the image pickup surface of the image pickup device 23.

That is, the image pickup device 23 and the A/D conversion section 25 which correspond to an image pickup section of the present embodiment generate an image including respective color components corresponding to the intensity of light received via the excitation light cut filter 24a and the R filter of the color filter 23a, and the intensity of light received via the excitation light cut filter 24a and the G filter of the color filter 23a in the fluorescence observation mode.

The color balance processing section 41 applies processing for adjusting a balance in the signal intensity among the red color component RD, the green color component GD and the blue color component BD included in the image data to the image data outputted from the A/D conversion section 25 and outputs the image data to the image processing unit 42.

Upon detecting that the endoscope apparatus 1 has been switched to the fluorescence observation mode based on the mode switching signal outputted from the mode switching control section 45, the image processing unit 42 operates so as to perform processing through the signal conversion section 42a and the matrix conversion section 42b.

The signal conversion section 42a performs processing of converting the red color component RD, the green color component GD and the blue color component BD included in the image data outputted from the color balance processing section 41 to a luminance component Y, and color difference components Cr and Cb.

The matrix conversion section 42b applies a matrix outputted from the storage section 27 of the scope 2 to the luminance component Y, and the color difference components Cr and Cb obtained as the processing result of the signal conversion section 42a.

The R filter, the G filter and the B filter of the color filter 23a have transmission characteristics over a wide band from a visible region to a near-infrared region respectively. For this reason, the component based on the wavelength component of the FL light received via the R filter of the color filter 23a and the component based on the wavelength component of the REF light received via the R filter of the color filter 23a are mixed with the red color component RD included in the image data outputted from the color balance processing section 41 in the fluorescence observation mode of the present embodiment. Furthermore, the component based on the wavelength component of the FL light received via the G filter of the color filter 23a and the component based on the wavelength component of the REF light received via the G filter of the color filter 23a are mixed with the green color component GD included in the image data outputted from the color balance processing section 41 in the fluorescence observation mode of the present embodiment. Therefore, if the red color component RD is allocated to the R channel of the display apparatus 5 just as is and the green color component GD is allocated to the G channel of the display apparatus 5 just as is, there is a problem that an observed image with an originally intended color tone is not displayed.

According to the present embodiment, the signal conversion section 42a and the matrix conversion section 42b perform processing of obtaining image data including only the red color component based on the wavelength component of the FL light received via the R filter of the color filter 23a and image data including only the green color component based on the wavelength component of the REF light received via the G filter of the color filter 23a before allocating the color components to the R, G and B channels of the display apparatus 5 as the processing to solve the aforementioned problem. The matrix calculation method or the like used for such processing will be described in detail below.

First, in a case where the R light and the G light are simultaneously emitted to a living tissue dispensed with the same fluorescent probe as that used for fluorescence observation of the site to be observed 101, the FL light and the REF light are received on the image pickup surface of the image pickup device 23 via the excitation light cut filter 24a and the color filter 23a, and image data I_{RGB} corresponding to the received FL light and REF light are generated by the A/D conversion section 25, a matrix corresponding to the intensity of the red color component RD, the green color component GD and the blue color component BD included in the image data I_{RGB} is defined as shown in following equation (1). In following equation (1), suppose R_{FL} represents the intensity of the red color component based on the wavelength component of the FL light received via the R filter of the color filter 23 a, G_{FL} represents the intensity of the green color component based on the wavelength component of the FL light received via the G filter of the color filter 23a, B_{FL} represents the intensity of the blue color component based on the wavelength component of the FL light received via the B filter of the color filter 23a, R_{REF} represents the intensity of the red color component based on the wavelength component of the REF light received via the R filter of the color filter 23a, G_{REF} represents the intensity of the green color component based on the wavelength component of the REF light received via the G filter of the color filter 23a, and B_{REF} represents the intensity of the blue color component based on the wavelength component of the REF light received via the B filter of the color filter 23a. ${I}_{RGB} = \left(\begin{matrix}{R}_{FL} & {R}_{REF} \\ {G}_{FL} & {G}_{REF} \\ {B}_{FL} & {B}_{REF}\end{matrix}\right)$

The image data I_{RGB} represented by the above equation (1) is converted to image data I_{YC} provided with a luminance color difference component expressed by following equation (2) through processing by the signal conversion section 42a. In following equation (2), suppose that Y_{FL} represents the magnitude of the luminance component in the wavelength component of the FL light, C_{RFL} and Cb_{FL} represent the magnitudes of the color difference components in the wavelength component of the FL light, Y_{REF} represents the magnitude of the luminance component in the wavelength component of the REF light, and Cr_{REF} and Cb_{REF} represent the magnitude of the color difference components in the wavelength component of the REF light. ${I}_{YC} = \left(\begin{matrix}{Y}_{FL} & {Y}_{REF} \\ {Cr}_{FL} & {Cr}_{REF} \\ {Cb}_{FL} & {Cb}_{REF}\end{matrix}\right)$

Here, when a matrix for separating image data of two mutually independent color components from the respective color components included in the image data outputted from the color balance processing section 41 is assumed to be MAT and a matrix representing the image data of the respective color components intended to be obtained as the processing result of the matrix conversion section 42b is assumed to be S, the processing involved in the separation of color components performed in the matrix conversion section 42b can be expressed by the following equations (3) and (4). $S = MAT ⋅ {I}_{YC}$ $S = \left(\begin{matrix}1 & 0 \\ 0 & 1\end{matrix}\right)$

By calculating following equation (5) based on above equations (3) and (4), a matrix MAT of 2 rows and 3 columns can be obtained. In following equation (5), suppose I_{YC}⁺ represents a pseudo-inverse matrix of I_{YC}. Furthermore, in the matrix S in (4), suppose the first row represents the output of the red color component, the second row represents the output of the green color component, the first column represents the output of the signal component of the FL light, and the second column represents the output of the signal component of the REF light. $MAT = S ⋅ {{I}_{YC}}^{+} = \left(\begin{matrix}M 11 & M 12 & M 13 \\ M 21 & M 22 & M 23\end{matrix}\right)$

According to the processing using the matrix MAT obtained through the calculation of equation (5) above, it is possible to separate image data including only the red color component FLRD based on the wavelength component of the FL light received via the excitation light cut filter 24a and the R filter of the color filter 23a from each color component included in the image data outputted from the color balance processing section 41.

Furthermore, according to the processing using the matrix MAT obtained through the calculation of equation (5) above, it is possible to separate image data including only the green color component REFGD based on the wavelength component of the REF light received via the excitation light cut filter 24a and the G filter of the color filter 23a from each color component included in the image data outputted from the color balance processing section 41.

According to the processing using the matrix MAT obtained through the calculation of equation (5) above, image data of the aforementioned red color component FLRD and the green color component REFGD can be obtained, whereas image data of the blue color component cannot be obtained. Therefore, in the present embodiment, a matrix MATA of 3 rows and 3 columns expressed as following equation (6) whose coefficients are set so as to be able to obtain image data of the blue color component REFBD having the same intensity as that of the image data of the aforementioned green color component REFGD is stored in the storage section 27 of the scope 2. Note that coefficients M11, M12, M13, M21, M22 and M23 in following equation (6) are assumed to have the same values as the respective coefficients included in the matrix MAT of 2 rows and 3 columns obtained through the calculation of equation (5) above. $MATA = \left(\begin{matrix}M 11 & M 12 & M 13 \\ M 21 & M 22 & M 23 \\ M 21 & M 22 & M 23\end{matrix}\right)$

That is, the storage section 27 of the scope 2 stores a matrix MATA for separating images expressed as equation (6) above calculated beforehand in accordance with the intensity of the respective color components included in the image data I_{RGB} generated (by the image pickup device 23 and the A/D conversion section 25) in the fluorescence observation mode.

The matrix conversion section 42b performs calculation by applying the matrix MATA for separating images outputted from the storage section 27 to the luminance component Y, and the color difference components Cr and Cb as a processing result of the signal conversion section 42a, and thereby acquires image data of the red color component FLRD having the intensity corresponding to coefficients M11, M12 and M13 expressed as following equation (7), image data of the green color component REFGD having the intensity corresponding to coefficients M21, M22 and M23 and image data of the blue color component REFBD having the intensity corresponding to coefficients M21, M22 and M23. $\left(\begin{matrix}FLRD \\ REFGD \\ REFBD\end{matrix}\right) = \left(\begin{matrix}M 11 ⋅ Y + M 12 ⋅ Cr + M 13 ⋅ Cb \\ M 21 ⋅ Y + M 22 ⋅ Cr + M 23 ⋅ Cb \\ M 21 ⋅ Y + M 22 ⋅ Cr + M 23 ⋅ Cb\end{matrix}\right)$

Furthermore, the matrix conversion section 42b allocates the image data of the red color component FLRD to the R channel of the display apparatus 5, allocates the image data of the green color component REFGD to the G channel of the display apparatus 5 and allocates the image data of the blue color component REFBD to the B channel of the display apparatus 5.

Then, the image processing unit 42 applies processing such as noise correction, gamma correction and edge enhancement to the image data allocated to the respective color channels of R, G and B of the display apparatus 5 through the processing by the matrix conversion section 42b, and outputs the image data to the D/A conversion section 43.

The display apparatus 5 displays an image of the object corresponding to the video signal outputted via the D/A conversion section 43.

That is, the above-described operation or the like is performed in the fluorescence observation mode, and an observed image (pseudo-color image) corresponding to the fluorescence observation mode is displayed on the display apparatus 5.

Note that according to the present embodiment, not only a matrix MATA specific to each scope 2 is stored in the storage section 27, but also ID information that can specify the type or the like of the scope 2 may be stored in the storage section 27, for example. In accordance with such a configuration of the storage section 27, the matrix conversion section 42b may have a configuration capable of selecting one matrix MATA corresponding to the ID information outputted from the storage section 27 from among a plurality of matrices MATA pre-stored in a memory (not shown) or the like.

Furthermore, according to the present embodiment, when acquiring measured values corresponding to each component of image data I_{RGB} or I_{YC} used to calculate the matrix MAT which forms the basis of the aforementioned matrix MATA, by appropriately adjusting the optical characteristics of the excitation light cut filter 24a, the wavelength bands of the R light and the G light emitted from the light source apparatus 3 and the fluorescence wavelength of the fluorescent probe (fluorescent substance) respectively and configuring (setting) the respective components so as to pick up an image of FL light and an image of REF light whose bands are narrowed as much as possible with brightness at which image pickup is possible, the values of the respective coefficients (M11, M12, M13, M21, M22 and M23) included in the matrices MAT and MATA can be optimized.

As described above, according to the present embodiment, it is possible to perform fluorescence observation with a configuration at lower cost and with wider applicability than the prior arts and also generate (display) an observed image during fluorescence observation with an originally intended color tone.

Note that the present invention is not limited to the aforementioned embodiment, and it goes without saying that various modifications and applications can be made without departing from the scope of the invention.

The present application is filed claiming the priority of Japanese Patent Application No. 2011-268190 filed in Japan on December 7, 2011.

## Claims

1. An endoscope apparatus for generating an image of a site to be observed (101) containing a fluorescent substance that is excited by excitation light in a red color region and emits fluorescence in a near-infrared region , comprising:
a light source section (3) configured to simultaneously emit to the site to be observed (101), excitation light in a red color region and reference light in a predetermined wavelength band which is a blue color region or a green color region for generating reflected light from the site to be observed (101);
an excitation light cut filter section (24a) configured to have optical characteristics that shut off the excitation light and allow light in a wavelength band other than the excitation light to substantially pass therethrough; **characterized by** a color filter section (23a) provided with a first filter configured such that transmittance in a region from the red color region to the near-infrared region is relatively higher than transmittance in other wavelength bands, and a second filter configured such that transmittance in the predetermined wavelength band of the reference light is relatively higher than the transmittance in the other wavelength bands;
an image pickup section (23) including pixels for generating an image pickup signal by picking up an image of light which is among return light emitted from the site to be observed according to emission of the excitation light and the reference light to the site to be observed (101) and which passed through the excitation light cut filter section (24a) and the first filter, and pixels for generating an image pickup signal by picking up an image of light which is among the return light and which passed through the excitation light cut filter section (24a) and the second filter; and
an image processing unit (42) configured to perform processing to acquire a first image obtained by separating a first color component corresponding to the fluorescence, image which is picked up after passing through the excitation light cut filter section (24a) and the first filter, from respective color components included in the image pickup signal generated by the image pickup section (23), and a second image obtained by separating a second color component corresponding to the reference light, image which is picked up after passing through the excitation light cut filter section (24a) and the second filter, from the respective color components.

2. The endoscope apparatus according to claim 1, further comprising:
an observation mode switching section (45) configured to output a mode switching signal corresponding to an instruction issued from a mode switching switch, to a filter switching control section (34), which is configured to issue a filter switching signal to a filter switching apparatus (24), to switch between a white light observation mode and a fluorescence observation mode; and the filter switching section (24) configured to retract, when the observation mode switching section (45) switches the mode to the white light observation mode, the excitation light cut filter (24a) from the optical path between an objective optical system of the endoscope and the color filter section (23a), and configured to insert, when the observation mode switching section (45) switches the mode to the fluorescence observation mode, the excitation light cut filter (24a) into the optical path between an objective optical system of the endoscope and the color filter section (23a).

3. The endoscope apparatus according to claim 1, further comprising a storage section (27) that stores a matrix for separating an image calculated beforehand in accordance with intensity of respective color components included in images generated by the image pickup section (23) when the excitation light and the reference light are simultaneously emitted to the site to be observed (101) including the fluorescent substance,
wherein the image processing unit (42) converts each color component included in each image pickup signal generated by the image pickup section to a luminance component and a color difference component, and further performs a calculation by applying the matrix for separating an image stored in the storage section (27) to the luminance component and the color difference component to thereby acquire the first image and the second image.

4. The endoscope apparatus according to claim 1, further comprising a signal conversion section (42a) and a matrix conversion section (42b), adapted to perform processing for obtaining image data including only the red color component based on the wavelength component of fluorescent light received via the first filter of the color filter (23a) and image data including only the green or blue color component based on the wavelength component of the reference light received via the second filter of the color filter (23a) before allocating the color components to the R, G and B channels of a display apparatus (5).

## Patentansprüche

1. Endoskopvorrichtung zum Erzeugen eines Bilds einer zu beobachtenden Stelle (101), enthaltend eine fluoreszierende Substanz, die durch Anregungslicht in einer roten Farbregion angeregt wird und Fluoreszenz in einer Nahinfrarotregion emittiert, umfassend:
einen Lichtquelleriabschnitt (3), der so konfiguriert ist, dass er gleichzeitig Anregungslicht in einer roten Farbregion und Referenzlicht in einem vordefinierten Wellenlängenband, das eine blaue Farbregion oder eine grüne Farbregion ist, an die zu beobachtende Stelle (101) emittiert, um reflektiertes Licht von der zu beobachtenden Stelle (101) zu erzeugen;
einen Anregungslichtsperrfilterabschnitt (24a), der so konfiguriert ist, dass er optische Charakteristika aufweist, die das Anregungslicht abschaltet und ermöglichen, dass Licht in einem Wellenlängenband, bei dem es sich nicht um das Anregungslicht handelt, diesen im Wesentlichen passieren;
**gekennzeichnet durch**
einen Farbfilterabschnitt (23a), der mit einem ersten Filter versehen ist, der so konfiguriert ist, dass eine Transmittanz in einer Region von der roten Farbregion zur Nahinfrarotregion relativ höher als eine Transmittanz in anderen Wellenlängenbändern ist, und mit einem zweiten Filter versehen ist, der so konfiguriert ist, dass eine Transmittanz im vordefinierten Wellenlängenband des Referenzlichts relativ höher als die Transmittanz in den anderen Wellenlängenbändern ist;
einen Bildaufnahmeabschnitt (23), umfassend Pixel zum Erzeugen eines Bildaufnahmesignals durch Aufnehmen eines Bilds von Licht, das zu zurückkommendem Licht gehört, das von der zu beobachtenden Stelle emittiert wird, gemäß einer Emission des Anregungslichts und des Referenzlichts zu der zu beobachtenden Stelle (101), und das den Anregungslichtsperrfilterabschnitt (24a) und den ersten Filter passiert hat, und Pixel zum Erzeugen eines Bildaufnahmesignals durch Aufnehmen eines Bilds von Licht, das zu dem zurückkommenden Licht gehört und das den Anregungslichtsperrfilterabschnitt (24a) und den zweiten Filter passiert hat; und
eine Bildverarbeitungseinheit (42), die so konfiguriert ist, dass sie eine Verarbeitung durchführt, um ein erstes Bild zu erfassen, das durch Trennen einer ersten Farbkomponente, die der Fluoreszenz entspricht, ein Bild, das nach Passieren des Anregungslichtsperrfilterabschnitts (24a) und des ersten Filters aufgenommen wird, von jeweiligen Farbkomponenten erhalten wird, die im Bildaufnahmesignal umfasst sind, das vom Bildaufnahmeabschnitt (23) erzeugt wird, und um ein zweites Bild zu erfassen, das durch Trennen einer zweiten Farbkomponente, die dem Referenzlicht entspricht, ein Bild, das nach Passieren des Anregungslichtsperrfilterabschnitts (24a) und des zweiten Filters aufgenommen wird, von den jeweiligen Farbkomponenten erhalten wird.

2. Endoskopvorrichtung nach Anspruch 1, die ferner umfasst:
einen Beobachtungsmodusschaltabschnitt (45), der so konfiguriert ist, dass er ein Modusschaltsignal, das einer Anweisung entspricht, die von einem Modusschaltschalter ausgegeben wird, an einen Filterschaltsteuerabschnitt (34) ausgibt, der so konfiguriert ist, dass er ein Filterschaltsignal an eine Filterschaltvorrichtung (24) ausgibt, um zwischen einem Weißlichtbeobachtungsmodus und einem Fluoreszenzbeobachtungsmodus zu schalten; und
wobei der Filterschaltabschnitt (24) so konfiguriert ist, dass er, wenn der Beobachtungsmodusschaltabschnitt (45) den Modus auf den Weißlichtbeobachtungsmodus schaltet, den Anregungslichtsperrfilter (24a) aus dem optischen Weg zwischen einem optischen Objektivsystem des Endoskops und dem Farbfilterabschnitt (23a) zurückzieht, und der so konfiguriert ist, dass er, wenn der Beobachtungsmodusschaltabschnitt (45) den Modus auf den Fluoreszenzbeobachtungsmodus schaltet, den Anregungslichtsperrfilter (24a) in den optischen Weg zwischen einem optischen Objektivsystem des Endoskops und dem Farbfilterabschnitt (23a) einsetzt.

3. Endoskopvorrichtung nach Anspruch 1, die ferner einen Speicherabschnitt (27) umfasst, der eine Matrix zum Trennen eines Bilds speichert, wie vorab berechnet, gemäß einer Intensität jeweiliger Farbkomponenten, die in Bildern umfasst sind, die vom Bildaufnahmeabschnitt (23) erzeugt wurden, wenn das Anregungslicht und das Referenzlicht gleichzeitig an die zu beobachtende Stelle (101) emittiert werden, umfassend die fluoreszierende Substanz,
wobei die Bildverarbeitungseinheit (42) jede Farbkomponente, die in jedem Bildaufnahmesignal umfasst ist, das vom Bildaufnahmeabschnitt erzeugt wird, in eine Luminanzkomponente und eine Farbunterschiedkomponente umwandelt, und ferner eine Berechnung durch Anwenden der Matrix zum Trennen eines im Speicherabschnitt (27) gespeicherten Bilds auf die Luminanzkomponente und die Farbunterschiedkomponente durchführt, um somit das erste Bild und das zweite Bild zu erfassen.

4. Endoskopvorrichtung nach Anspruch 1, die ferner einen Signalumwandlungsabschnitt (42a) und einen Matrixumwandlungsabschnitt (42b) umfasst, die so ausgelegt sind, dass sie eine Verarbeitung durchführen, um Bilddaten zu erhalten, die nur die rote Farbkomponente umfassen, auf Basis der Wellenlängenkomponente von fluoreszierendem Licht, das über den ersten Filter des Farbfilters (23a) empfangen wird, und Bilddaten zu erhalten, die nur die grüne oder blaue Farbkomponente umfassen, auf Basis der Wellenlängenkomponente des Referenzlichts, das über den zweiten Filter des Farbfilters (23a) empfangen wurde, bevor die Zuordnung der Farbkomponenten zu den R-, G- und B-Kanälen einer Anzeigevorrichtung (5) erfolgt.

## Revendications

1. Appareil d'endoscope pour générer une image d'un site à observer (101) contenant une substance fluorescente qui est excitée par une lumière d'excitation dans une région de couleur rouge et émet une fluorescence dans une région de proche infrarouge, comprenant :
une section de source de lumière (3) configurée pour émettre simultanément, vers le site à observer (101), une lumière d'excitation dans une région de couleur rouge et une lumière de référence dans une bande de longueur d'onde prédéterminée qui est une région de couleur bleue ou une région de couleur verte pour générer une lumière réfléchie depuis le site à observer (101) ;
une section de filtre de coupure de lumière d'excitation (24a) configurée pour avoir des caractéristiques optiques qui coupent la lumière d'excitation et autorisent la lumière dans une bande de longueur d'onde autre que la lumière d'excitation de passer de manière substantielle à travers celle-ci ;
une section de filtre coloré (23a) comportant un premier filtre configuré de telle sorte qu'un facteur de transmission dans une région allant de la région de couleur rouge à la région de proche infrarouge est relativement plus élevé qu'un facteur de transmission dans d'autres bandes de longueur d'onde, et un second filtre configuré de telle sorte qu'un facteur de transmission dans la bande de longueur d'onde prédéterminée de la lumière de référence est relativement plus élevé que le facteur de transmission dans les autres bandes de longueur d'onde ;
une section de capture d'image (23) comprenant des pixels pour générer un signal de capture d'image en capturant une image de lumière qui est parmi une lumière de retour émise depuis le site à observer selon l'émission de la lumière d'excitation et de la lumière de référence vers le site à observer (101) et qui est passée à travers la section de filtre de coupure de lumière d'excitation (24a) et le premier filtre, et des pixels pour générer un signal de capture d'image en capturant une image de lumière qui est parmi la lumière de retour et qui est passée à travers la section de filtre de coupure de lumière d'excitation (24a) et le second filtre ; et
une unité de traitement d'image (42) configurée pour réaliser un traitement pour acquérir une première image obtenue en séparant une première composante de couleur correspondant à la fluorescence, image qui est capturée après passage à travers la section de filtre de coupure de lumière d'excitation (24a) et le premier filtre, à partir de composantes de couleur respectives comprises dans le signal de capture d'image généré par la section de capture d'image (23), et une seconde image obtenue en séparant une seconde composante de couleur correspondant à la lumière de référence, image qui est capturée après passage à travers la section de filtre de coupure de lumière d'excitation (24a) et le second filtre, à partir des composantes de couleur respectives.

2. Appareil d'endoscope selon la revendication 1, comprenant en outre :
une section de commutation de mode d'observation (45) configurée pour émettre un signal de commutation de mode, correspondant à une instruction délivrée par un commutateur de commutation de mode, vers une section de commande de commutation de filtre (34), qui est configurée pour délivrer un signal de commutation de filtre à un appareil de commutation de filtre (24), pour commuter entre un mode d'observation de lumière blanche et un mode d'observation de fluorescence ; et
la section de commutation de filtre (24) configurée pour rétracter, lorsque la section de commutation de mode d'observation (45) commute le mode sur le mode d'observation de lumière blanche, le filtre de coupure de lumière d'excitation (24a) vis-à-vis du trajet optique entre un système optique d'objectif de l'endoscope et la section de filtre coloré (23a), et configurée pour insérer, lorsque la section de commutation de mode d'observation (45) commute le mode sur le mode d'observation de fluorescence, le filtre de coupure de lumière d'excitation (24a) dans le trajet optique entre un système optique d'objectif de l'endoscope et la section de filtre coloré (23a).

3. Appareil d'endoscope selon la revendication 1, comprenant en outre une section de stockage (27) qui stocke une matrice pour séparer une image calculée au préalable conformément à l'intensité de composantes de couleur respectives comprises dans des images générées par la section de capture d'image (23) lorsque la lumière d'excitation et la lumière de référence sont émises simultanément vers le site à observer (101) comprenant la substance fluorescente,
l'unité de traitement d'image (42) convertissant chaque composante de couleur comprise dans chaque signal de capture d'image généré par la section de capture d'image en une composante de luminance et une composante de différence de couleur, et réalisant en outre un calcul en appliquant la matrice pour séparer une image stockée dans la section de stockage (27) à la composante de luminance et à la composante de différence de couleur, pour acquérir ainsi la première image et la seconde image.

4. Appareil d'endoscope selon la revendication 1, comprenant en outre une section de conversion de signal (42a) et une section de conversion de matrice (42b), aptes à réaliser un traitement pour obtenir des données d'image comprenant uniquement la composante de couleur rouge sur la base de la composante de longueur d'onde de la lumière fluorescente reçue par l'intermédiaire du premier filtre du filtre coloré (23a) et des données d'image comprenant uniquement la composante de couleur verte ou bleue sur la base de la composante de longueur d'onde de la lumière de référence reçue par l'intermédiaire du second filtre du filtre coloré (23a) avant d'attribuer les composantes de couleur aux canaux R, V et B d'un appareil d'affichage (5).
